(19) Europäisches Patentamt
European Patent Office
Office européen des brevets

(11) Numéro de publication: **0 131 526**
**B1**

(12) # FASCICULE DE BREVET EUROPEEN

(45) Date de publication du fascicule du brevet: **20.11.86**

(51) Int. Cl.[4]: **C 07 C 89/00**, C 07 C 91/44

(21) Numéro de dépôt: **84420092.3**

(22) Date de dépôt: **21.05.84**

(54) **Procédé de préparation d'amino-phénols par hydroxylation d'anilines en milieu superacide au moyen du péroxyde d'hydrogène.**

(30) Priorité: **29.06.83 FR 8310730**

(43) Date de publication de la demande: **16.01.85 Bulletin 85/3**

(45) Mention de la délivrance du brevet: **20.11.86 Bulletin 86/47**

(84) Etats contractants désignés: **BE DE FR GB IT NL**

(56) Documents cités:
**EP - A - 0 027 593**
**EP - A - 0 041 441**
**EP - A - 0 097 564**
**FR - A - 2 318 851**

**JOURNAL OF ORGANIC CHEMISTRY, volume 46, no. 21, 9 octobre 1981, G.A. OLAH et al.: "Oxyfunctionalization of hydrocarbons. II. Hydroxylation of benzene and alkylbenzenes with hydrogen peroxide in hydrogen fluoride/boron trifluoride", pages 4305-4306**

**Le dossier contient des informations techniques présentées postérieurement au dépôt de la demande et ne figurant pas dans le présent fascicule.**

(73) Titulaire: **ATOCHEM, 4 & 8, Cours Michelet La Défense 10, F-92800 Puteaux (FR)**

(72) Inventeur: **Morellet, Guy, 32 rue des Carmes, F-86000 Poitiers (FR)**
Inventeur: **Jacquesy, Jean-Claude, 46 rue du Planty Buxerolles, F-86000 Poitiers (FR)**
Inventeur: **Jouannetaud, Marie-Paule, 8 Allée du Nivernais, F-86000 Poitiers (FR)**

**EP 0 131 526 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention concerne un procédé de préparation d'amino-phénols par hydroxylation en milieu superacide liquide, au moyen du peroxyde d'hydrogène, d'amines aromatiques de formule générale:

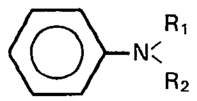

dans laquelle $R_1$ et $R_2$ représentent soit un atome d'hydrogène, soit un radical alkyle contenant de 1 à 8 atomes de carbone. Les amines aromatiques utilisables sont donc:

— l'aniline, amine primaire
— les N-alkylanilines, amines secondaires
— les N,N-dialkylanilines, amines tertiaires.

Le procédé objet de l'invention permet d'introduire sur le noyau aromatique de l'amine une fonction phénol dans les trois positions ortho, méta e para par rapport au groupement amine, sans entraîner de réaction parasites importantes d'oxydation de la fonction azotée. La proportion de méta-aminophénol formé est toujours importante, voire prépondérante.

Dans sa demande de brevet français N° 82 10644 la demanderesse a décrit un procédé d'hydroxylation par le peroxyde d'hydrogène, en milieu superacide, des anilides, c'est-à-dire des amides dérivés de l'aniline. Il est particulièrement surprenant qu'un procédé analogue puisse être appliqué à des anilines non-protégées, car l'art antérieur enseigne très clairement que les composés peroxydiques (peroxyde d'hydrogène, peracides organiques, hydroperoxydes, peroxydes de diacyle) attaquent en général l'azote de la fonction amine, avec formation d'hydroxylamine, d'oxyde d'amine, de dérivés nitrosés, azo, azoxy ou de polymères. On observe parfois une attaque des groupements alkyles portés par l'azote des amines aromatiques secondaires et tertiaires, entraînant des réactions de désalkylation ou la formation d'imines.

Ces différents modes d'action des composés peroxydiques sur les amines aromatiques sont décrits notamment dans les références suivantes:

— B.C. CHALLIS et A.R. BUTLER, in «The Chemistry of the Amino Group» (PATAI), p. 320-338, Interscience Publishers, New-York, 1968.

— M. HEDAYATULLAH, Bull. Soc. Chim. France, *1972*, p. 2957-2974.

— J.P.SCHIRMANN et S.Y. DELAVARENNE, «Hydrogen Peroxide in Organic Chemistry», chapitre VII, p. 147-156, EDI Paris, 1979.

Dans le cas particulier des N,N-dialkylanilines, l'oxydation par des ions peroxydisulfate, connue sous le nom de réaction de BOYLAND-SIMS, conduit à l'introduction sur le noyau aromatique d'un groupement sulfate, hydrolysable ultérieurement en groupement phénol. Cette introduction se fait essentiellement en position ortho par rapport au groupement amine, l'isomère para ne se formant en proportions significatives que lorsque les deux positions ortho sont bloquées. Il ne se forme pas d'isomère méta. Cette réaction de BOYLAND-SIMS a été étudié notamment par E.J. BEHRMAN et D.M. BEHRMAN, J. Org. Chem., *43*, n° 23, p. 4551-4552 (1978).

Le brevet européen publié sous le n° 0 041 441 décrit un procédé d'hydroxylation préférentielle en position méta par rapport à la fonction phénol des alkyl-phénols et de leurs éthers en milieu superacide au moyen du peroxyde d'hydrogène. Mais le but visé n'est atteint qu'à cause de la présence d'un ou de plusieurs substituants alkyls sur le noyau aromatique des phénols ou de leurs éthers, présence dont sont privées les amines à hydroxyler concernées par l'invention.

L'hydroxylation sur le noyau aromatique des amines libres est réalisée, selon la présente invention, en faisant agir à basse température le peroxyde d'hydrogène sur l'amine désirée au sein d'un milieu superacide liquide.

Ce milieu superacide liquide est constitué de produits qui, sur l'échelle logarithmique de HAMMETT, ont des acidités atteignant environ —25. A titre de comparaison, l'acide sulfurique à 100% a une acidité de —11 et l'acide fluorhydrique anhydre une acidité de —10. Les superacides liquides utilisés ont donc des acidités jusqu'à $10^{14}$ fois plus élevées que celles des acides minéraux forts habituels. Comme exemples de superacides liquides utilisables dans le procédé selon l'invention, on peut citer «l'acide magique» F $SO_3H$ - $SbF_5$, l'acide fluoroantimonique HF - $SbF_5$, les complexes de l'acide trifluorométhanesulfonique avec le pentafluorure d'antimoine $CF_3$ $SO_3$ H - $SbF_5$ et le complexe de l'acide fluorhydrique avec le trifluorure de bore HF - $BF_3$.

Le peroxyde d'hydrogène est mis en oeuvre sous forme de solutions aqueuses commerciales, dont le titre peut aller de 20 à 98% en poids. On utilise préférentiellement la solution commerciale titrant 70% en poids de $H_2O_2$. Le rapport molaire du peroxyde d'hydrogène au substrat à hydroxyler est généralement compris entre 1 et 2.

La proportion de superacide par rapport à l'amine aromatique peut varier dans de larges limites. Elle est en général d'autant plus élevée que la solution aqueuse de peroxyde d'hydrogène est plus diluée. Avec une solution de peroxyde d'hydrogène à 70% en poids on utilise habituellement de 4 à 20 millilitres de superacide par millimole de substrat.

La réaction se déroule à des températures allant de —50°C environ à 0°C environ, à la pression atmosphérique ou sous des pressions supérieures à la pression atmosphérique, notamment lorsqu'on utilise les complexes HF - $BF_3$. La durée de réaction va de quelques minutes à une heure.

Le mode opératoire général suivi pour mettre en œuvre le procédé selon l'invention est très simple: dans un récipient en poly(tétrafluoréthylène), ou en acier inoxydable lorsqu'on opère avec les complexes HF - $BF_3$, muni d'une agitation magnétique et refroidi à la température désirée, on introduit successivement le superacide liquide, l'amine aromatique et le peroxyde d'hydrogène. Le mélange est maintenu sous agitation pendant le temps voulu à la température choisie. Il est ensuite versé sur un mélange d'eau, de glace et de carbonate acide de sodium.

Après neutralisation, la solution est extraite plusieurs fois par un solvant organique léger, tel que l'éther diéthylique. Après évaporation du solvant d'extraction, le produit brut est chromatographié sur gel de silice, en utilisant comme éluant un solvant organique ou un mélange de solvents organiques permettant la séparation des produits de réaction.

Selon une variante de ce mode opératoire général, on peut introduire le peroxyde d'hydrogène dans le superacide avant le substrat à hydroxyler. Selon d'autres variantes, convenant notamment pour les mélanges HF - $BF_3$, on introduit dans le réacteur d'abord le substrat, puis le superacide et le peroxyde d'hydrogène, ou le substrat, puis le peroxyde d'hydrogène et le superacide.

Les exemples suivants, donnés à titre non-limitatif, illustrent divers aspects de la mise en œuvre du procédé selon l'invention.

*Exemple 1*

Hydroxylation de l'aniline en milieu HF - $SbF_5$.

a) Suivant le mode opératoire général, on refroidit à —40°C un mélange de 17,5 ml d'acide fluorhydrique anhydre et de 2,5 ml de pentafluorure d'antimoine (rapport molaire $SbF_5/HF$ = 0,04). On lui ajoute successivement 4 millimoles d'aniline et 4 millimoles de proxyde d'hydrogène sous forme d'une solution aqueuse à 70% en poids. Le mélange réactionnel est maintenu sous agitation pendant 15 minutes à —40°C.

Après traitement, le produit brut obtenu, pesant 320 mg, est chromatographié sur gel de silice sous pression d'azote. Un mélange 50/50 en volumes d'hexane et d'acétate d'éthyle élue successivement l'aniline non transformée, l'amino-2-phénol, l'amino-3-phénol et l'amino-4-phénol. La structure des produits formés découle de leurs caractéristiques physiques (point de fusion) et spectroscopiques (résonance magnétique nucléaire, infra-rouge et spectre de masse) et aussi de leur identification à des produits connus.

b) On répète l'essai ci-dessus, mais à la température de —20°C et en utilisant un rapport molaire $H_2O_2$/substrat de 1,5 au lieu de 1,0.

Les résultats quantitatifs de ces deux essais sont donnés dans le tableau 1.

TABLEAU 1

| Essai | Taux de transformation de l'aniline | Rendement en aminophénols | Proportions relatives des isomères | | |
|---|---|---|---|---|---|
| | | | ortho | méta | para |
| 1 a | 68% | 40% | 35 | 57 | 8 |
| 1 b | 95% | 70,5% | 29 | 51 | 20 |

*Exemple 2*

Hydroxylation de la N,N-diméthylaniline en milieu HF - $SbF_5$ par le peroxyde d'hydrogène à 70%.

On refroidit à —20°C un mélange de 15,75 ml d'acide fluorhydrique anhydre et de 2,25 ml de pentafluorure d'antimoine (rapport molaire $SbF_5/HF$ = 0,04) et on lui ajoute successivement 4 millimoles de N,N-diméthylaniline et 4 millimoles de peroxyde d'hydrogène sous forme d'une solution aqueuse à 70% en poids.

Le mélange réactionnel est maintenu à —20°C sous agitation pendant 15 minutes. Après traitement, on obtient 540 mg de produit brut, qui est chromatographié sur gel de silice sous pression d'azote. Un mélange 70/30 en volumes d'hexane et d'acétate d'éthyle permet d'éluer successivement:

— la N,N-diméthylaniline non transformée (12%)

— l'hydroxy-2-N,N-diméthylaniline (14%)

— l'hydroxy-3-N,N-diméthylaniline (44%)

— l'hydroxy-4-N,N-diméthylaniline (26%)

Les proportions relatives des isomères hydroxylés sont donc:

— ortho . . . . . . . . . . . . 17%

— méta . . . . . . . . . . . . 52%

— para . . . . . . . . . . . . 31%

L'isomère para étant difficilement identifiable par résonane magnétique nucléaire du proton, il a été caractérisé par le spectre RMN de son dérivé acétylé, obtenu par action de l'anhydride acétique.

*Exemple 3*

Hydroxylation de la N,N-diéthylaniline en milieu HF/$SbF_5$.

a) A un mélange de 15,75 ml d'acide fluorhydrique anhydre et de 2,25 ml de pentafluorure d'antimoine (rapport molaire $SbF_5/HF$ = 0,04), refroidi à —20°C, on ajoute successivement 4 millimoles de N,N-diéthylaniline et 4,4 millimoles de peroxyde d'hydrogène sous forme d'une solution aqueuse à 70% en poids. Le mélange réactionnel est maintenu sous agitation à —20°C pendant 15 minutes.

Après traitement, le produit brut, pesant 610 mg, est chromatographié sur gel de silice. Le mélange 90/10 en volumes d'hexane et d'éther diéthylique élue 390 mg d'un mélange composé de N,N-diéthylaniline (312 mg) non transformée et d'hydroxy-2-N,N-diéthylaniline (78 mg). Le dosage des deux produits présents dans le mélange est effectué par RMN du proton.

Le mélange 70/30 en volumes d'hexane et d'éther diéthylique élue 190 mg d'hydroxy-3-N,N-diéthylaniline.

Enfin le mélange 60/40 en volumes d'hexane et d'éther diéthylique élue 22 mg d'un mélange de deux produits qui n'ont pas été parfaitement identifiés. Cependant le spectre de masse du mélange fait apparaître un pic qui correspondrait à l'hydroxy-4-N,N--diéthylaniline.

b) On répète l'essai ci-dessus, mais en utilisant un rapport molaire $H_2O_2$/substrat de 1,5 au lieu de 1,1.

Dans cet essai, le mélange des deux produits les plus polaires, élué par le mélange 60/40 hexane/éther diéthylique, pèse 140 mg.

Le tableau 2 rassemble les résultats quantitatifs des deux essais 3 a et 3 b.

Le rendement en hydroxy-4-N,N-diéthylaniline, insuffisamment caractérisée, n'est pas indiqué.

TABLEAU 2

| Essai | Taux de transformation de la N,N-diéthylaniline | Rendements en dérivés hydroxylés | | |
|---|---|---|---|---|
| | | ortho | méta | para |
| 3 a | 48% | 12% | 29% | — |
| 3 b | 87,5% | 11% | 49% | — |

*Exemple 4*

Hydroxylation de la N-éthylaniline en milieu HF - $SbF_5$.

On refroidit à —20°C un mélange de 17,5 ml d'acide fluorhydrique anhydre et de 2,5 ml de pentafluorure d'antimoine (rapport molaire $SbF_5/HF$ = 0,04) et on lui ajoute successivement 4 millimoles de N-éthylaniline et 6 millimoles de peroxyde d'hydrogène sous forme d'une solution aqueuse à 70% en poids.

La durée de réaction sous agitation à —20°C est de 15 minutes.

Après traitement on obtient 520 mg de produit brut, qui est chromatographié sur gel de silice.

Le mélange 85/15 en volumes d'hexane et d'éther diéthylique élue 23 mg (4,5%) de N-éthylaniline n'ayant pas réagi.

Le mélange 70/30 en volumes d'hexane et d'éther diéthylique élue 100 mg (18%) d'hydroxy-2-N-éthylaniline.

Le mélange 65/35 en volumes d'hexane et d'éther diéthylique élue 221 mg (40,5%) d'hydroxy-3-N-éthylaniline.

Le mélange 55/45 en volumes d'hexane et d'éther diéthylique élue 90 mg (16,5%) d'hydroxy-4-N-éthylaniline.

Les proportions relatives des isomères hydroxylés sont donc:

— ortho . . . . . . . . . . . . 24%
— méta . . . . . . . . . . . . 54%
— para . . . . . . . . . . . . 22%

*Exemple 5*

Hxdroxylation de la N,N-diméthylaniline en milieu HF - $SbF_5$ par le peroxyde d'hydrogène à 35%.

a) A un mélange de 17,5 ml d'acide fluorhydrique anhydre et de 2,5 ml de pentafluorure d'antimoine (rapport molaire $SbF_5/HF$ = 0,04), refroidi à —20°C, on ajoute successivement 2 millimoles de N,N-diméthylaniline et 3 millimoles de peroxyde d'hydrogène sous forme d'une solution aqueuse à 35% en poids.

Après 15 minutes de réaction à —20°C sous agitation, le mélange réactionnel est traité de la manière habituelle.

On obtient 240 mg de produit brut, qui est chromatographié sur gel de silice. La N,N-diméthylaniline non transformée est éluée par un mélange hexaneéther diéthylique 90/10 en volumes. L'hydroxy-2-N,N-diméthylaniline est éluée par un mélange 80/20 des mêmes solvants. Le mélange 70/30 élue l'hydroxy-3-N,N-diméthylaniline et le mélange 55/45 élue l'hydroxy-4-N,N-diméthylaniline. Un mélange 45/55 permet d'éluer 15 mg d'un produit plus polaire, qui n'a pas été identifié.

b) On répète l'essai précédent, mais en utilisant un mélange de 7,5 ml d'acide fluorhydrique et de 2,5 ml de pentafluorure d'antimoine (rapport molaire $SbF_5/HF$ = 0,09) et en mettant en réaction 3,4 millimoles de peroxyde d'hydrogène à 35%.

On obtient après traitement 270 mg de produit brut, qui est chromatographié comme ci-dessus. Le mélange 45/55 hexane/éther diéthylique élue 25 mg d'un produit non identifié.

Le tableau 3 donne les résultats quantitatifs des essais 5 a et 5 b.

TABLEAU 3

| Essai | Taux de transformation de la N,N-diméthylaniline | Rendement en diméthylaminophénols | Proportions relatives des isomères | | |
|---|---|---|---|---|---|
| | | | ortho | méta | para |
| 5 a | 54,5% | 26,5% | 11 | 60,5 | 28,5 |
| 5 b | 65% | 57,5% | 12 | 63 | 25 |

*Exemple 6*

Hydroxylation de la N,N-diméthylaniline en milieu $CF_3SO_3H$ - $SbF_5$.

a) On homogénéise à —20°C un mélange de 6 ml d'acide trifluorméthanesulfonique et de 3 ml de pentafluorure d'antimoine (rapport molaire $SbF_5/CF_3SO_3H$ = 0,6), et on lui ajoute successivement 3 millimoles de proxyde d'hydrogène sous forme d'une solution aqueuse à 70% en poids et 2 millimoles de N,N-diméthylaniline. Le mélange réactionnel est maintenu à —20°C sous agitation pendant 8 minutes.

Après traitement, on obtient 125 mg de produit brut. La chromatographie de ce produit, effectuée comme dans l'exemple 5, permet d'obtenir 40 mg (rendement 14,5%) d'hydroxy-3-N,N-diméthylaniline et 40 mg (rendement 14,5%) d'hydroxy-4-N,N-diméthylaniline.

b) On répète l'essai précédent, mais en introduisant dans le milieu superacide l'amine avant le peroxyde d'hydrogène et en portant la durée de réaction à 10 minutes.

On obtient 160 mg de produit brut. La chromatographie permet de récupérer 38% de N,N-diméthylaniline non transformée et d'isoler 48 mg (rendement 17,5%) d'hydroxy-3-N,N-diméthylaniline et 20 mg (rendement 7,5%) d'hydroxy-4-N,N-diméthylani-

line. Le mélange 45/55 hexane/éther diéthylique élue 19 mg d'un produit plus polaire non identifié.

*Exemple 7*

Hydroxylation de la N,N-diméthylaniline en milieu $FSO_3H$ - $SbF_5$.

On refroidit à —20°C un mélange de 6 ml d'acide fluorosulfurique et de 3 ml de pentafluorure d'antimoine (rapport molaire $SbF_5/FSO_3H = 0,4$), et on lui ajoute successivement 3 millimoles de peroxyde d'hydrogène sous forme de solution aqueuse à 70% en poids et 2 millimoles de N,N-diméthylaniline. La durée de réaction sous agitation à —20°C est de 15 minutes.

On obtient après traitement 160 mg de produit brut, qui est filtré sur gel de silice. Le mélange 70/30 en volumes d'hexane et d'éther diéthylique élue 62 mg (rendement 22,5%) d'hydroxy-3-N,N-diméthylaniline, tandis que le mélange 55/45 des mêmes solvants élue 45 mg (rendement 16,5%) d'hydroxy-4--N,N-diméthylaniline. Le reste du produit brut est constitué de matières polymères non identifiées.

*Exemple 8*

Hydroxylation de la N,N-diméthylaniline en milieu HF - $BF_3$.

Dans un récipient en poly(tetrafluoréthylène) refroidi à —40°C, on verse 15 ml d'acide fluorhydrique anhydre, dans lesquels on fait barboter pendant 15 minutes un courant de trifluorure de bore. On ajoute ensuite 2 millimoles de N,N-diméthylaniline, puis 2,2 millimoles de peroxyde d'hydrogène (solution aqueuse à 70% en poids). On fait barboter à nouveau un courant de trifluorure de bore et maintient le milieu sous agitation à —40°C pendant 20 minutes.

Après traitement on obtient 250 mg de produit brut, qui est chromatographié sur gel de silice. Le mélange 85/15 en volumes hexane/éther diéthylique élue la N,N-diméthylaniline non transformée (210 mg - 87%). Le mélange 70/30 des mêmes solvants élue 16 mg (6%) d'hydroxy-3-N,N-diméthylaniline. Il ne s'est formé que moins de 1% de dérivé parahydroxylé.

## Revendications

1. Procédé de préparation d'amino-phénols, donnant une proportion élevée d'isomère méta, caractérisé en ce que l'on fait réagir le peroxyde d'hydrogène dans un milieu liquide superacide, à des températures allant d'environ —50°C à environ 0°C, sur des amines aromatiques non protégées, répondant à la formule générale:

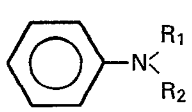

dans laquelle $R_1$ et $R_2$ représentent soit un atome d'hydrogène, soit un radical alkyle contenant de 1 à 8 atomes de carbone.

2. Procédé selon la revendication 1, caractérisé en ce que le milieu superacide liquide est constitué par un complexe d'acide fluorhydrique anhydre et de pentafluorure d'antimoine.

3. Procédé selon la revendication 1, caractérisé en ce que le milieu superacide liquide est constitué par un complexe d'acide fluorosulfonique et de pentafluorure d'antimoine.

4. Procédé selon la revendication 1, caractérisé en ce que le milieu superacide liquide est constitué par un complexe d'acide trifluorométhanesulfonique et de pentafluorure d'antimoine.

5. Procédé selon la revendication 1, caractérisé en ce que le milieu superacide liquide est constitué par un complexe d'acide fluorhydrique anhydre et de trifluorure de bore.

6. Procédé selon l'une quelconque des revendications 1 à 5, caractérisé en ce que le peroxyde d'hydrogène est mis en œuvre sous forme d'une solution aqueuse contenant de 20 à 98% en poids de peroxyde d'hydrogène.

7. Procédé selon l'une quelconque des revendications 1 à 6, caractérisé en ce que le rapport molaire du peroxyde d'hydrogène à l'amine aromatique est compris entre 1 et 2.

8. Procédé selon l'une quelconque des revendications 1 à 7, caractérisé en ce que l'on utilise de 4 à 20 millilitres de superacide liquide par millimole de substrat à hydroxyler.

## Patentansprüche

1. Verfahren zur Herstellung von Aminophenolen mit einem erhöhten Anteil an Meta-Isomeren, dadurch gekennzeichnet, dass man in einem flüssigen superaziden Medium bei Temperaturen zwischen etwa —50°C und 0°C Wasserstoffperoxid mit nicht geschützten aromatischen Aminen der allgemeinen Formel

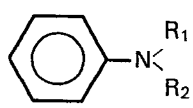

umsetzt, in der $R_1$ und $R_2$ ein Wasserstoffatom oder ein Alkylradikal mit 1 bis 8 Kohlenstoffatomen bedeuten.

2. Verfahren nach Anspruch 1, dadurch gekennzeichnet, dass das flüssige superazide Milieu durch einen Komplex aus wasserfreiem Fluorwasserstoff und Antimonpentafluorid gebildet wird.

3. Verfahren nach Anspruch 1, dadurch gekennzeichnetz, dass das flüssige superazide Milieu durch einen Komplex aus Fluorsulfonsäure und Antimonpentafluorid gebildet wird.

4. Verfahren nach Anspruch 1, dadurch gekennzeichnet, dass das flüssige superazide Milieu durch einen Komplex aus Trifluormethansulfonsäure und Antimonpentafluorid gebildet wird.

5. Verfahren nach Anspruch 1, dadurch gekennzeichnet, dass das flüssige superazide Milieu durch einen Komplex aus wasserfreier Fluorwasserstoffsäure und Bortrifluorid gebildet wird.

6. Verfahren nach einem der Ansprüche 1 bis 5, dadurch gekennzeichnet, dass das Wasserstoffperoxid in Form einer wässrigen Lösung mit 20 bis 98 Gewichtsprozent Wasserstoffperoxid eingesetzt wird.

7. Verfahren nach einem der Ansprüche 1 bis 6, dadurch gekennzeichnet, dass das Molverhältnis zwischen Wasserstoffperoxid und dem aromatischen Amin zwischen 1 und 2 liegt.

8. Verfahren nach einem der Ansprüche 1 bis 7, dadurch gekennzeichnet, dass man 4 bis 20 Milliliter flüssiger Supersäure pro Millimol zu hydroxylierendem Substrat einsetzt.

**Claims**

1. Process for the preparation of aminophenols, yielding a high proportion of the meta isomer, characterized in that hydrogen peroxide is reacted in a superacid liquid medium, at temperatures ranging from approximately —50°C to approximately 0°C, with unprotected aromatic amines corresponding to the general formula:

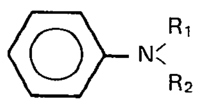

in which $R_1$ and $R_2$ denote either a hydrogen atom or an alkyl radical containing from 1 to 8 carbon atoms.

2. Process according to Claim 1, characterized in that the liquid superacid medium consists of a complex of anhydrous hydrofluoric aicd with antimony pentafluoride.

3. Process according to Claim 1, characterized in that the liquid superacid medium consists of a complex of fluorosulphonic acid with antimony pentafluoride.

4. Process according to Claim 1, characterized in that the liquid superacid medium consists of a complex of trifluoromethanesulphonic acid with antimony pentafluoride.

5. Process according to Claim 1, characterized in that the liquid superacid medium consists of a complex of anhydrous hydrofluoric acid with boron trifluoride.

6. Process according to any one of Claims 1 to 5, characterized in that hydrogen peroxide is used in the form of an aqueous solution containing from 20 to 98% by weight of hydrogen peroxide.

7. Process according to any one of Claims 1 to 6, characterized in that the molar ratio of hydrogen peroxide to the aromatic amine is between 1 and 2.

8. Process according to any one of Claims 1 to 7, characterized in that from 4 to 20 millilitres of liquid superacid are used per millimole of the substrate to be hydroxylated.